(19) **Europäisches Patentamt**
**European Patent Office**
**Office européen des brevets**

(11) **EP 1 385 500 B1**

(12) **EUROPEAN PATENT SPECIFICATION**

(45) Date of publication and mention
of the grant of the patent:
**28.07.2010 Bulletin 2010/30**

(51) Int Cl.:
*A61K 31/22* (2006.01)   *A61K 31/23* (2006.01)
*A61K 31/231* (2006.01)   *A61K 31/045* (2006.01)
*A61K 31/215* (2006.01)   *A61K 31/25* (2006.01)

(21) Application number: **02724588.5**

(22) Date of filing: **11.04.2002**

(86) International application number:
**PCT/IL2002/000294**

(87) International publication number:
**WO 2002/083122 (24.10.2002 Gazette 2002/43)**

(54) **FATTY ALCOHOLS AND FATTY ACID ESTERS USEFUL FOR TREATMENT OF INFLAMMATION**

FETTALKOHOLE UND FETTSÄUREESTER ZUR BEHANDLUNG VON ENTZÜNDUNGEN

ALCOOLS GRAS ET ESTERS D'ACIDES GRAS UTILES DANS LE TRAITEMENT D'INFLAMMATION

(84) Designated Contracting States:
**AT BE CH CY DE DK ES FI FR GB GR IE IT LI LU MC NL PT SE TR**
Designated Extension States:
**AL LT LV MK RO SI**

(30) Priority: **11.04.2001 IL 14253501**

(43) Date of publication of application:
**04.02.2004 Bulletin 2004/06**

(73) Proprietor: **YEDA RESEARCH AND DEVELOPMENT CO., LTD.**
**76100 Rehovot (IL)**

(72) Inventors:
• **COHEN, Irun, R.**
**76354 Rehovot (IL)**
• **SHINITZKY, Meir**
**46910 Kfar Shmaryahu (IL)**
• **MARGALIT, Raanan**

**(IL)**

(74) Representative: **Vossius & Partner**
**Siebertstrasse 4**
**81675 München (DE)**

(56) References cited:
**WO-A-01/00139      WO-A-99/04632**
**WO-A-02/083122    US-A- 3 592 930**
**US-A- 5 194 451    US-B1- 6 210 700**
**US-B1- 6 280 755    US-B1- 6 331 568**
**US-B1- 6 365 628**

• SNIPES W ET AL: "Inactivation of lipid-containing viruses by long-chain alcohols." ANTIMICROBIAL AGENTS AND CHEMOTHERAPY. JAN 1977, vol. 11, no. 1, January 1977 (1977-01), pages 98-104, XP009034119 ISSN: 0066-4804
• SANDS J ET AL: "EXTREME SENSITIVITY OF ENVELOPED VIRUSES, INCLUDING HERPES SIMPLEX, TO LONG-CHAIN UNSATURATED MONOGLYCERIDES AND ALCOHOLS" ANTIMICROBIAL AGENTS AND CHEMOTHERAPY, AMERICAN SOCIETY FOR MICROBIOLOGY, WASHINGTON, DC, US, vol. 15, no. 1, January 1979 (1979-01), pages 67-73, XP009007372 ISSN: 0066-4804

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD OF THE INVENTION**

The present invention relates to anti-inflammatory agents and, more particularly, to fatty alcohols, esters thereof with $C_1$ - $C_6$ alkanoic acids or esters of fatty acids with alkanediols or glycerol which are useful in the treatment of immunologically-mediated inflammation.

**Abbreviations: AA:** adjuvant arthritis: **CFA:** complete Freund's adjuvant; **EAE:** experimental autoimmune encephalomyelitis; **GPSCH:** guinea pig spinal cord homogenate; **IFA:** incomplete Freund's adjuvant; **OA:** oleyl alcohol; **PBS:** phosphate-buffered saline; **SC:** subcutaneously.

**BACKGROUND OF THE INVENTION**

Inflammation is commonly divided into three phases: acute inflammation, the immune response and chronic inflammation. Acute inflammation is the initial response to tissue injury and is mediated by the release of histamine, serotonin, bradykinin, prostaglandins and leukotrienes. The immune response, usually preceded by the acute inflammation phase, occurs when immunologically competent cells are activated in response to foreign organisms or antigenic substances liberated during the acute or chronic inflammatory response. The outcome of the immune response for the host may be beneficial, as when it causes invading organisms to be phagocytosed or neutralized. However, the outcome may be deleterious if it leads to chronic inflammation without resolution of the underlying injurious process as it occurs in rheumatoid arthritis.

The treatment of patients with inflammation envisages the relief of pain, which is the presenting symptom and the major continuing complaint of the patient, as well as the slowing or arrest of the tissue-damaging process.

Anti-inflammatory agents are usually classified as steroidal or glucocorticoids and nonsteroidal anti-inflammatory agents (NSAIDs). The glucocorticoids are powerful anti-inflammatory agents but the high toxicity associated with chronic corticosteroid therapy inhibits their use except in certain acute inflammatory conditions. Therefore, the nonsteroidal anti-inflammatory drugs have assumed a major role in the treatment of chronic conditions such as rheumatoid arthritis.

Among the nonsteroidal anti-inflammatory agents are included derivatives of aminoarylcarboxylic acids, arylacetic acids, arylbutyric acids, arylcarboxylic acids, arylpropionic acids, pyrazole, pyrazolone, salicylic acid and some other derivatives of different chemical structure, including specific anti-arthritic/anti-rheumatic agents.

Some fatty alcohols and esters of fatty acids have been described as solvents or emulsifiers for use in pharmaceutical compositions. For example, cetyl alcohol may be used in pharmaceutical compositions as emulsifying and stiffening agent (The Merck Index, pp. 347-8, # 2037), oleyl alcohol may be used as a carrier for medicaments (The Merck Index, p. 1222, # 6900), and alkyl esters of oleic acid may be used as solvents for medicaments (The Merck Index, p. 6899, # 6898).

A mixture of higher aliphatic primary alcohols, primarily isolated from beeswax, was described as having moderate anti-inflammatory activity. The composition of such a mixture was not disclosed (Rodriguez et al., 1998).

Feeding laboratory animals with fish oil rich in the long-chain n-3 polyunsaturated fatty acids (PUFAs), eicosapentaenoic acid (20:5n-3) and docosahexaenoic acid (22:6n-3), was described to reduce acute and chronic inflammatory responses, to improve survival to endotoxin and in models of autoimmunity and to prolong the survival of grafted organs, and it was therefore suggested that fish oil supplementation may be clinically useful in acute and chronic inflammatory conditions and following transplantation (Calder, 1998). A pharmaceutical preparation comprising eicosapentaenoic acid and/or stearidonic acid for treatment of schizophrenia is described in WO 98/16216 and US 6,331,568.

Modified polyunsaturated fatty acids and derivatives thereof have been proposed for pharmaceutical uses. WO 99/27924 and US 6,280,755 describe anti-inflammatory fatty acids uninterrupted by a methylene group for use in topical pharmaceutical and cosmetic compositions. WO 97/38688 and US 6,262,119 describe polyunsaturated fatty acids having 1 or 2 substitutions selected from oxa and thia in position beta or gamma to the acyl group, for treating or ameliorating symptoms of T-cell mediated disease. WO 99/58122 and US 6,365,628 describe saturated fatty acids in which one or more methylene groups are substituted by O, S, SO, $SO_2$, or Se and alkyl esters thereof, for treatment or prevention of diabetes. US 5,019,383 describes synthetic vaccines comprising a peptide residue coupled to one or more alkyl or alkenyl groups of at least 12 carbon atoms or other lipophilic substance, wherein said alkyl or alkenyl group may be a fatty acid residue coupled to one or more functional groups of a polyfunctional group which is bound to the N-terminal amino group and/or C-terminal carboxy group of the peptide residue.

There is no description in the literature that isolated fatty alcohols or esters thereof with alkanoic acids may be used themselves as medicaments, and specifically not that they may be involved in immunomodulation of inflammation.

## SUMMARY OF THE INVENTION

It has now been surprisingly found, in accordance with the present invention, that certain long-chain fatty alcohols, esters thereof with $C_1$ - $C_6$ alkanoic acids, or certain esters of long-chain fatty acids with alkanediols or glycerol can suppress inflammation in experimental adjuvant arthritis (AA) and experimental autoimmune encephalomyelitis (EAE) models in rats and can prevent graft rejection in mice.

The present invention thus relates to pharmaceutical compositions for the treatment of inflammation, particularly immunologically-mediated inflammation, comprising as active ingredient an immunomodulator selected from: (a) a saturated or cis-unsaturated $C_{10}$-$C_{18}$ fatty alcohol.

In another embodiment, the invention relates to the use of an immunomodulator selected from: (a) a saturated or cis-unsaturated $C_{10}$- $C_{18}$ fatty alcohol for the preparation of a pharmaceutical composition for the treatment of inflammation, in particular immunologically-mediated inflammation.

## BRIEF DESCRIPTION OF THE DRAWINGS

Fig. 1 shows the dose response effect of oleyl alcohol (OA) on adjuvant arthritis (AA). Different doses of OA were administered subcutaneously to rats once 14 days before induction of AA.

Fig. 2 is a graph showing the disease profile of Lewis rats with experimental autoimmune encephalomyelitis (EAE) and treated with oleyl alcohol. Oleyl alcohol was administered to the rats 14 days before induction of EAE. Control group was treated with incomplete Freund's adjuvant (IFA).

Fig. 3 is a graph showing the disease profile of Lewis rats with EAE and treated with IFA. IFA was administered to the rats 14 days before induction of EAE. Control group was not treated.

## DETAILED DESCRIPTION OF THE INVENTION

The present invention provides immunomodulators selected from: a saturated or cis-unsaturated $C_{10}$- $C_{18}$ fatty alcohol.

According to one preferred embodiment of the invention, the pharmaceutical composition comprises a long-chain saturated or unsaturated $C_{10}$-$C_{18}$, preferably $C_{16}$-$C_{18}$, most preferably a $C_{18}$, fatty alcohol.

Examples of $C_{10}$-$C_{18}$ saturated fatty alcohols that can be used according to the invention include, but are not limited to, decyl alcohol, lauryl alcohol, myristyl alcohol, stearyl alcohol and preferably cetyl alcohol (also known as palmityl alcohol).

The unsaturated fatty alcohol according to the invention has preferably one or more double bonds in the cis form and 16-18 carbon atoms and may be, without being limited to, oleyl alcohol (cis-9-octadecenol), linoleyl alcohol (cis-9,12-octadecadienol), $\gamma$-linolenyl alcohol (cis-6,9,12-octadecatrienol) and linolenyl alcohol (cis-9,12,15-octadecatrienol). In preferred embodiments, the fatty alcohol used in the compositions of the invention is cetyl, linolenyl or, most preferably, oleyl alcohol.

The $C_{10}$-$C_{18}$ fatty acid is preferably a $C_{16}$-$C_{18}$ most preferably a $C_{18}$ fatty acid. In one embodiment, the $C_{10}$-$C_{18}$ fatty acid is saturated such as, but without being limited to, capric acid, lauric acid, myristic acid, palmitic acid, stearic acid and arachidic acid. In another embodiment, the $C_{10}$-$C_{20}$ fatty acid is a cis-unsaturated fatty acid such as, but without being limited to, palmitoleic acid (cis-9-hexadecenoic acid), oleic acid (cis-9-octadecenoic acid), cis-vaccenic acid (cis-11-octadecenoic acid), linoleic acid (cis-9,12-octadecadienoic acid), $\gamma$-linolenic acid (cis-6,9,12-octadecatrienoic acid), linolenic acid (cis-9,12,15-octadecatrienoic acid) and arachidonic acid (cis-5,8,11,14-eicosatetraenoic acid).

According to the invention, the alkanediol has 2 to 8, preferably 2 to 4, and more preferably, 2 carbon atoms, and is selected from, but not being limited to, 1,3-propanediol, 1,4-butanediol and, preferably, 1,2-ethylene glycol. An example of such an ester is 1,2-ethylene glycol monooleate.

According to another embodiment of the invention, the active ingredient of the pharmaceutical composition is a mono- or diester of glycerol with the long-chain fatty acid. In one preferred embodiment, the monoglyceride is glycerol monooleate. The diglycerides contain one free hydroxyl group and the other two hydroxyl groups may be both esterified with 2 molecules of the long-chain fatty acid, e.g. glycerol dioleate, or one of the hydroxyl groups is esterified with one molecule of the long-chain fatty acid and a second hydroxyl group is esterified with a $C_1$- $C_6$ alkanoic acid such as acetic acid, propionic acid, butyric acid, valeric acid and caproic acid.

The immune system, in both its innate and adaptive arms, is involved in regulating inflammation of every type, and inflammation is a key factor in processes such as wound healing, connective tissue re-modeling, angiogenesis, organ regeneration, neuroprotection, as well as in the adaptive immune responses seen in autoimmunity, allergies, graft rejection, and infection (see Cohen, 2000; Schwartz and Cohen, 2000). Therefore, anti-inflammatory agents that modulate the inflammatory response such as those described here will be useful in a variety of conditions.

Inflammatory disorders that can be treated with the immunomodulators of the present invention include, but are not limited to, immunologically-mediated chronic or acute inflammatory disorders selected from an autoimmune disease, severe allergies, asthma, graft rejection or for the treatment of chronic degenerative diseases such as Alzheimer's disease, and in neuroprotection, organ regeneration, chronic ulcers of the skin, and schizophrenia.

Examples of autoimmune diseases that can be treated according to the invention are multiple sclerosis or a human arthritic condition, e.g. rheumatoid arthritis, reactive arthritis with Reiter's syndrome, ankylosing spondylitis and other inflammations of the joints mediated by the immune system. Other autoimmune diseases are contemplated and are presented in the following list in the context of the organ or tissue involved. Thus, according to the invention, the immunologically-mediated inflammatory disorder may be myasthenia gravis, Guillain-Barré syndrome, and other inflammatory diseases of the nervous system; psoriasis, pemphigus vulgaris and other diseases of the skin; systemic lupus erythematosus, glomerulonephritis and other diseases affecting the kidneys; atherosclerosis and other inflammations of the blood vessels; autoimmune hepatitis, inflammatory bowel diseases, e.g. Crohn's disease, pancreatitis, and other conditions of the gastrointestinal system; type 1 diabetes mellitus (insulin-dependent diabetes mellitus or IDDM), autoimmune thyroiditis (Hashimoto's thyroiditis), and other diseases of the endocrine system.

One of the models used to test the anti-inflammatory activity of the agents according to the invention is adjuvant arthritis (AA), an experimental disease of the joints inducible in some strains of rats by immunizing with *Mycobacterium tuberculosis* in complete Freund's adjuvant (CFA). These animals develop an arthritis whose features are similar to those of rheumatoid arthritis in humans and thus serve as animal models of human arthritic conditions such as rheumatoid arthritis, reactive arthritis in Reiter's syndrome, ankylosing spondylitis and other inflammations of the joints which appear to be mediated by the immune system (Pearson, 1964). Adjuvant arthritis also serves as a model of immune-mediated inflammation in general including cell-mediated autoimmune reactions, graft rejection and allergic reaction. For example, treatments which can suppress rheumatoid arthritis include immunosuppressive agents such as corticosteroids, cyclosporin A (Jaffee et al., 1989; Pollock et al., 1989), azathioprine, and other immunosuppressive agents which are broadly used in the treatment of autoimmune diseases. Therefore, suppression of adjuvant arthritis by a therapeutic agent indicates that the agent is potentially useful as a broad anti-inflammatory agent.

The pharmaceutical composition provided by the present invention may be in solid, semisolid or liquid form and may further include pharmaceutically acceptable fillers, carriers or diluents, and other inert ingredients and excipients. The composition can be administered by any suitable route such as, but not limited to, oral, topical, or parenteral e.g. by injection through subcutaneous, intravenous, intramuscular, or any other suitable route. Since many of the compounds are oily, they are preferably administered parenterally, more preferably subcutaneously. If given continuously, the compounds of the present invention are each typically administered by 1-4 injections per day or by continuous subcutaneous infusions, for example, using a mini-pump. The dosage will depend of the state of the patient and severity of the disease and will be determined as deemed appropriate by the practitioner.

For parenteral administration, the compounds may be formulated by mixing each at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation. Generally, the formulations are prepared by contacting the compounds of the present invention each uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils can be also useful, as well as liposomes. These preparations can be made by conventional methods known to those skilled in the art, for example as described in "Remington's Pharmaceutical Science", A.R. Gennaro, ed., 17th edition, 1985, Mack Publishing Company, Easton, PA, USA.

The invention will now be illustrated by the following non-limiting examples.

## EXAMPLES

### Example 1. Anti-inflammatory effect of oleyl alcohol and other agents-protection against adjuvant arthritis (AA)

AA was induced by immunizing inbred 8-10-week old Lewis rats (Harlan-Olac Limited, Blackthorn, Oxon, UK), at the base of the tail with 1 mg/0.1 ml of killed *Mycobacterium tuberculosis* (Sigma) in IFA (Sigma) as described (Pearson, 1956). Arthritis of the limbs was noted to develop 12-14 days later and was scored on a scale of 0-16 summing the severity of the inflammation of each of the 4 limbs on a scale of 0-4, as described (Holoshitz et al., 1983). The peak of the arthritis usually was observed around day 26 after immunization.

Control rats were untreated or treated by injections of saline. A positive control of immunosuppression was obtained by including a group of rats treated with the corticosteroid agent dexamethasone (200 $\mu$g) administered intraperitoneally every other day beginning on day 12 after induction.

The immunomodulator of the invention (100 μl oleyl alcohol, glycerol mono-oleate, linolenyl alcohol or cetyl alcohol) was administered subcutaneously (SC) once 14 days before induction of AA or on day 12 after induction of AA. The percent inhibition of inflammation measured on the day of maximal inflammation was computed as follows:

$$\frac{\text{mean maximal score of test group}}{\text{mean maximal score of control group}} \quad \text{x } 100\%$$

All four compounds were found to be effective, producing more than 60% inhibition of inflammation whereas oleic acid had no effect. The results are summarized in Table 1.

Two further experiments showed that 500 μl of oleyl alcohol (100 μl corresponds to about 90 mg oleyl alcohol) suppressed the inflammation by 96% and 91%.

**Table 1. Effects of various agents on the inflammation of adjuvant arthritis**

| Compound Tested | % Inhibition (100 μl) |
|---|---|
| Glycerol mono-oleate | 98% |
| Oleyl alcohol | 78% |
| Linolenyl alcohol | 75% |
| Cetyl alcohol | 66% |

**Example 2. Protection against AA by different doses of oleyl alcohol**

To study the dose response effect of oleyl alcohol in AA, oleyl alcohol was administered subcutaneously in doses of 10, 50,100 or 500 mg to Lewis rats once 14 days before induction of AA, as described in Example 1 above.

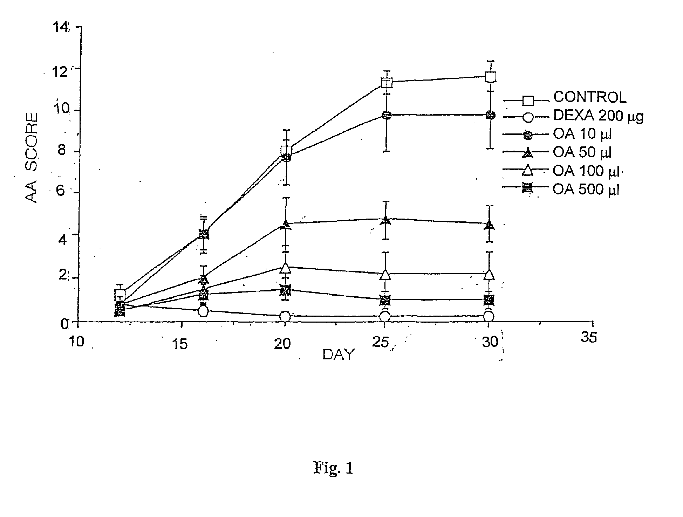

Fig. 1 shows the dose response effect of oleyl alcohol. It can be seen that increasing doses of oleyl alcohol suppressed the arthritis. On the day of peak disease, day 26, the inflammation was suppressed by 14% (10 μl), 61% (50 μl), 78% (100 μl) and 90% (500 μl).

**Example 3. Anti-inflammatory effect of oleyl alcohol and other immunomodulators and protection against EAE in DA rats**

Experimental autoimmune encephalomyelitis (EAE) is an experimental autoimmune disease inducible in some strains of rats by immunization with myelin basic protein (MBP) or proteolipid protein (PLP) in complete Freund's adjuvant (CFA) or with an emulsion of the rat's spinal cord in either CFA or incomplete Freund's adjuvant (IFA). EAE in DA rats is considered as a model of chronic EAE. Within two to three weeks the animals develop cellular infiltration of the myelin sheaths of the central nervous system resulting in demyelination and paralysis. Most of the animals die, but others have milder symptoms, and some animals develop a chronic form of the disease that resembles chronic relapsing and remitting multiple sclerosis (MS) in humans. Therefore, these animals with EAE serve as a model for the human MS autoimmune disease. EAE develops in the animal about 12 days after immunization and is characterized by paralysis of various degrees due to inflammation of the central nervous system. In some strains, like the Lewis rat, the paralysis can last up to 6-7 days and the rats usually recover unless they die during the peak of their acute paralysis. In other strains of rats like the DA rat, the paralysis can be chronic and remitting.

For the induction and clinical assessment of EAE, spinal cord obtained from DA rats is frozen, thawed and minced thoroughly with a spatula before immunization. Rats are immunized by one subcutaneous injection (just under the skin) into the dorsal base of the tail with 200 μl emulsion prepared from 1:1 IFA (Difco, Detroit, MI, USA) and antigen (volume/weight, i.e. 100 μl IFA/100 mg of whole spinal cord) or from 1:1 CFA (IFA was complemented with 4 mg/ml of *Mycobacterium tuberculosis* strain 37RA) and antigen (volume/weight, i.e. 100 μl CFA/100 mg of whole spinal cord). The emulsion was prepared by titration with a gas-tight glass syringe and a needle, 1.2 mm diameter. Rats are regularly weighed and examined for clinical signs of EAE. A four-graded scale was used to assess clinical severity: 0, no paralysis; 1, tail weakness (hanging); 2, hind limb paralysis; 3, hind and fore limb paralysis; 4, severe total paralysis (Lorentzen et al., 1995).

Groups of 5 or 7 DA strain female rats, 8-9 week old, are immunized in the hind footpads with 0.1 ml per footpad of IFA containing 100 mg of whole, homogenized DA spinal cord, for a total of 200 mg per rat. On the day of immunization, the rats are treated by SC injection with oleyl alcohol or other agent according to the invention (100 μl) or with paraffin

oil (control). The rats are scored for EAE on a severity scale of 0 - 4 as described above.

**Example 4. Anti-inflammatory effect of oleyl alcohol and protection against EAE in Lewis rats**

EAE induced in Lewis rats is considered as a model of acute inflammation in the brain (as opposed to the chronic disease in DA rats).

For EAE induction, three lyophilized guinea pig spinal cord homogenate (GPSCH) emulsions were prepared as follows: (i) 25 mg of lyophilized GPSCH (GP2) was suspended in 2.5 ml of sterile PBS (Sigma) and incubated for one hour at 37° C; (ii) 54.1 mg of *Mycobacterium tuberculosis* H37Ra (MT, Difco) was suspended in 13.5 ml CFA (Sigma) containing 1mg/ml MT to obtain 5 mg/ml MT; (iii) 2.5 ml CFA (5 mg/ml MT) was added into vial with 2.5 ml of PBS containing 25 mg GPSCH to yield 5 mg/ml GPSCH and 2.5 mg/ml MT. The mixture was transferred into a glass syringe connected to a second glass syringe through a Luer lock bridge. The material was mixed well by transferring from one syringe to another for about 10 minutes until the materiall was well emulsified. The emulsion of GPSCH at a dose of 1 mg/rat and MT at a dose of 0.5 mg/rat in CFA induced EAE in rats (based on previous titration).

For the treatment, two groups of eight 9-10 weeks old Lewis rats (Harlan, Israel), were treated with the test samples (oleyl alcohol or IFA) 14 days before EAE induction. The group treated with IFA served as the control group. The test samples were injected at a dose of 0.5 ml/kg once, subcutaneously. A third group of 8 rats was not treated and served as non-treated control.

EAE was induced in rats of all three groups 14 days after injection of the test samples by injection with 0.1 ml of the GPSCH emulsion in CFA into each of the hind leg foot pads (0.2 ml per rat).

The EAE clinical signs were observed and scored from the 9th day post-EAE induction until the termination of the experiment according to the following five-graded scale to assess clinical severity: 0, normal behavior; 1, weight loss; 2, tail weakness; 3, hind legs hypotonia and weakness; 4, hind legs paralysis; 4, severe total paralysis; 5, impaired respiration and/or convulsions and/or full paralysis or death. All rats having scores of 1 and above were considered sick.

The calculation of EAE results was carried out as follows:

(i) *Calculation of the incidence of disease*

The number of sick animals in each group were summed. The incidence of disease and the % activity were calculated as follows:

$$\text{Incidence of disease} = \frac{\text{No. of sick rats in group}}{\text{No. of rats in group}} \times 100\%$$

$$\% \text{ activity *} = 1 - \frac{(\text{disease incidence in treated group})}{\text{disease incidence in control group}} \times 100\%$$

$$* = (\text{according to incidence})$$

(ii) *Calculation of the mean maximal score (MMS)*

The maximal score of each rat in the group were summed. The mean maximal score (MMS) and the % activity of the group were calculated as follows:

$$\text{Mean Maximal score} = \frac{\sum \text{Maximal score of each rat}}{\text{No. of rats in the group}}$$

$$\% \text{ activity } * = (1 - \frac{\text{MMS of treated group}}{\text{MMS of control group}}) \times 100$$

$$* = (\% \text{ activity according to MMS})$$

(iii) *Calculation of the group mean score (GMS)*

The mean score of each rat during the observation period were summed (score 5 was counted forward). The mean score of the group and its % activity were calculated as follows:

$$\text{Mean score} = \frac{\sum \text{Group score of each rat}}{\text{No. of rats in the group}}$$

$$\% \text{ activity } * = (1 - \frac{\text{GMS of treated group}}{\text{GMS of control group}}) \times 100$$

$$* = (\% \text{ activity according to GMS})$$

(iv) *Calculation of the mean onset of disease*

The time of disease onset (days) for each rat in the group were summed. The mean onset of disease for the group was calculated. The time of onset of disease for those rats that did not develop EAE was considered as 25 days (duration of study).

(v) *Calculation of the mean duration of disease*

The disease duration (days) of each rat in each group were summed. The mean disease duration of the group was calculated. The disease duration of rats that did not develop EAE was considered as zero.

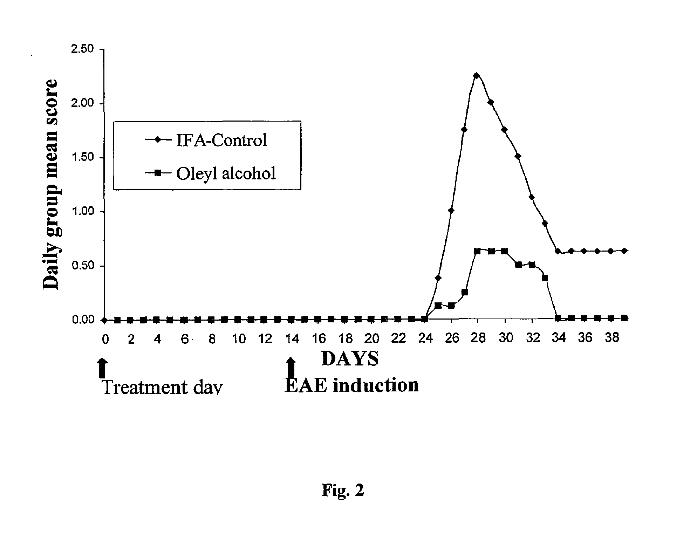

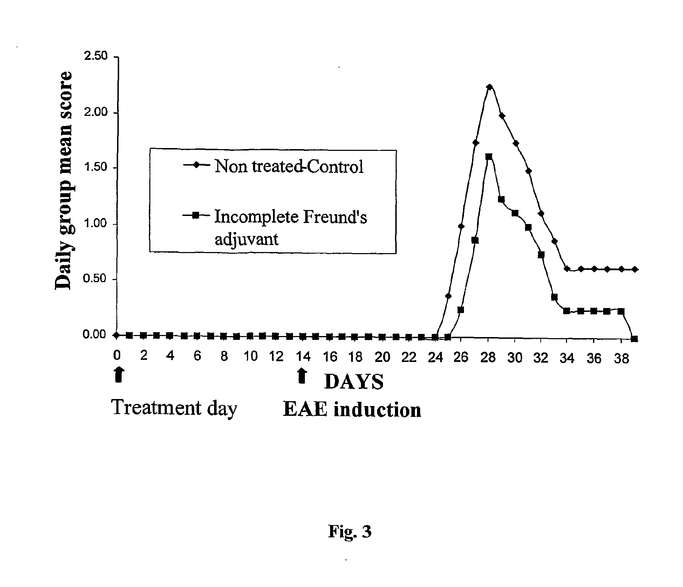

The evaluation of the clinical manifestations of EAE, i.e. % incidence of disease, MMS, GMS, mean duration and onset of EAE disease is summarized in Table 2. The graphs of the disease profile for each group are presented in Figs. 2 and 3 for treatment with oleyl alcohol and IFA, respectively.

As shown by the results, no essential difference in incidence of disease (62.5% to 75% incidence) or mean maximum score (1.75 to 2.38 MMS) was observed between the IFA-injected groups and non-treated control group. Oleyl alcohol showed a beneficial effect on all the clinical parameters that were tested. It exhibited 77.1% activity according to group mean score (GMS) and 63% activity according to mean maximum score (MMS) compared to the non-treated control group. The mean onset of disease was 18.6 days in the oleyl alcohol treated group compared to 15.5 days in the non-treated control group. The duration of disease was 2.0 days in the oleyl alcohol treated group compared to 5.13 days in the non-treated control group. The duration of the EAE clinical signs in the tested groups was between 1 and 7 days, except one rat in the group treated with IFA. IFA had minor effect, if any, on the rat EAE. No mortality was observed in the tested groups, except one rat in the non-treated control group.

**Table 2: Evaluation EAE clinical results**

| Group No. | Test Sample | % Incidence | % Activity Incidence | MMS | % Activity MMS | GMS | % Activity GMS | Mean Onset of disease (Day No.) | Disease duration (days) |
|---|---|---|---|---|---|---|---|---|---|
| 1 | OA | 50.0% | 33.3% | 0.88 | 63.0% | 0.22 | 77.1% | 18.6 | 2.0 |
| 2 | IFA | 62.5% | 16.7% | 1.75 | 26.5% | 0.52 | 45.8% | 17.0 | 3.75 |
| 3 | NTC | 75.0% | NA | 2.38 | NA | 0.96 | NA | 15.5 | 5.13 |
| OA-Oleyl alcohol; NTC-non-treated control; NA- Not applicable | | | | | | | | | |

## Example 5. Effect of oleyl alcohol on skin allograft survival

The immune system represents a strong barrier for successful transplantation of organs or tissues between non-genetically identical donor and recipient. Both CD4[+] and CD8[+] T cells participate in graft rejection.

Skin graft transplantation is carried out essentially as described before (Birk et al., 1999). Thus, mice are shaved and 1 cm$^2$ sections of skin are cut from the dorsal side of sacrificed donors and cleaned in PBS. Two patches of dorsal skin, 1 cm$^2$ each, are cut from the anesthetized recipients (Nembutal 6 mg/ml, 0.25 ml/mouse) in preparation for the allograft. Two donor allografts per recipient are grafted onto the dorsal lesioned patches. Histoacryl (B. Braun Melsungen AG, Melsungen, Germany) is applied around the graft. Nobecutan (ASTR, Astra Tech, Glos G15, UK) is sprayed over the grafts.

In the experiment, groups of 6 BALB/c female mice, 8-week old, are grafted with 1 cm$^2$, full thickness skin grafts from C57BL/6 female mice, 8-week old. On the day of grafting, a group of recipient mice is treated either with paraffin oil or SC with 100 $\mu$l oleyl alcohol or another immunomodulator according to the invention. The day of rejection is scored. The transplanted skin in the mice treated with the immunomodulator survives longer in comparison with the untreated control mice.

## Example 6. Prevention and treatment of SLE

Systemic lupus erythematosus is an autoimmune disease in which both autoantibodies and immune complexes are involved. In order to test the immunomodulators of the invention, mice with experimental SLE or (NZBxNZW)F1 mice that spontaneously develop autoimmune diseases that closely resemble SLE, can be used.

In order to induce experimental SLE, BALB/c mice are immunized with the human or murine anti-DNA monoclonal antibody 16/6Id (20 $\mu$g/mouse) in CFA in the hind footpads and boosted 3 weeks later with the same amount of the immunizing antibody in PBS. The mice are then tested for autoantibody production and clinical manifestations characteristic of experimental SLE. In order to either prevent induction of experimental SLE or to cure mice afflicted with the disease, mice are given oleyl alcohol or another immunomodulator according to the invention subcutaneously (100 $\mu$l per mouse) before or concomitant with the immunization and some weeks after immunization. The number of injections is based on the effect of the tested compound on the disease induction and progression. The animals are regularly weighed and examined for clinical signs of SLE as described, for example, in WO 96/30057.

## Example 7. Prevention and treatment of autoimmune thyroiditis

Experimental autoimmune thyroiditis (EAT) can be induced in a number of animals by immunizing with thyroglobulin in CFA. Both humoral antibodies and $T_{DTH}$ cells directed against the thyrogllobulin develop, resulting in thyroid inflammation. EAT appears to best mimic Hashimoto's thyroiditis.

EAT is induced as previously described (Rose et al., 1971) by injecting each mouse subcutaneously with thyroglobulin extract obtained from one thyroid gland. The extract is emulsified in IFA (Difco Laboratories, Detroit, Mich.), to which are added 7mg/ml *Mycobacterium tuberculosis,* H37Ra strain (Difco Laboratories). This injection is repeated one week later. Donors of thyroglobulin extract are mice of the C3H/eB strain. 4-5 weeks later, EAT is assayed by removing thyroid glands of recipient mice, fixing them in 10% formalin solution and then in 70% alcohol, and examining microscopic sections stained with hematoxylin and eosin. Microscopic slides are coded and examined without knowledge of their identity. A diagnosis of EAT is made by observing at least one unequivocal focus of infiltration by mononuclear cells. Treatment is performed by injecting SC oleyl alcohol or another immunomodulator (100 $\mu$l per animal) before induction of EAT, concomitant with or thereafter (control animals are injected paraffin oil), and the animals are regularly weighed and examined for clinical signs of EAT by known conventional methods.

**REFERENCES**

Birk et al., 1999. The 60kDa heat shock protein modulates allograft rejection. Proc. Nat. Acad. Science USA. 96: 5159-63.

Calder, PC. 1998. Immunoregulatory and anti-inflammatory effects of n-3 polyunsaturated fatty acids. Braz. J. Med. Biol. Res. 31:467-90.

Cohen, IR. 2000, Tending Adam's Garden: Evolving the Cognitive Immune Self. Academic Press, London, UK.

Cohen, IR. 2000. "Discrimination and Dialogue in the Immune System". Seminars in Immunology. 12: 215-219; 269-271; 321-323.

Holoshitz, Y. et al., 1983. "Lines of T lymphocytes mediate or vaccinate against autoimmune arthritis", Science 219: 56.

Jaffee, B.D. et al., 1989, "The effect of immunomodulating drugs on adjuvant-induced arthritis in Lewis rats", Agents Actions. 27 (3-4): 344-6.

Lorentzen J.C. et al., 1995, "Protracted, relapsing and demyelinating experimental autoimmune encephalomyelitis in DA rats immunized with syngeneic spinal cord and incomplete Freund's adjuvant", J. Neuroimmunology. 63: 193-205.

Pearson, CM. 1964, "Experimental models in rheumatoid disease", Arthritis Rheum. 7:80.

Pearson, CM. 1956, "Development of arthritis, periarthritis and periostitis in rats given adjuvant", Proc. Soc. Exp. Biol. Med. 91:91.

Pollock et al., 1989, "Pharmacokinetic analysis of cyclosporine in adjuvant arthritic rats", Drug Metab. Dispos. 17 (6): 595-9.

Rodriguez, M.D., Gamez, R., Sanchez, M and H. Garcia. 1998, J. Appl. Toxicol. 18: 313-6.

Rose, N.R., F.J. Twarog and A.J. Crowe. 1971. Murine thryoiditis: importance of adjuvant and mouse strain for the induction of thyroid lesions. J. Immunol. 106:698)

Schwartz, M. and Cohen.IR. 2000, "Autoimmunity can benefit self-maintenance". Immunol Today. 21:265-8.

The Merck Index, 13th Edition, 2001, Merck & Co. Inc., Rahway, N.J., U.S.A.

**Claims**

1. A saturated or cis-unsaturated $C_{10}$-$C_{18}$ fatty alcohol for use as an immunomodulator in the treatment of immunologically-mediated chronic or acute inflammatory disorders selected from an autoimmune disease, severe allergies, asthma, graft rejection or for the treatment of chronic degenerative diseases such as Alzheimer's disease, and in neuroprotection, organ regeneration, chronic ulcers of the skin, and schizophrenia.

2. The embodiment according to claim 1, wherein the immunomodulator is a saturated $C_{10}$-$C_{18}$ fatty alcohol such as decyl alcohol, lauryl alcohol, myristyl alcohol, cetyl alcohol and stearyl alcohol.

3. The embodiment according to claim 1, wherein the immunomodulator is a cis-unsaturated $C_{16}$-$C_{18}$ fatty alcohol such as oleyl alcohol, linoleyl alcohol, $\gamma$-linolenyl alcohol and linolenyl alcohol.

**4.** The embodiment according to claim 1, wherein said autoimmune disease is multiple sclerosis or a human arthritic condition.

**5.** The embodiment according to claim 4, wherein said human arthritic condition is selected from rheumatoid arthritis, reactive arthritis with Reiter's syndrome, ankylosing spondylitis and other inflammations of the joints mediated by the immune system.

**6.** The embodiment according to claim 1, wherein said immunologically-mediated inflammatory disorder is myasthenia gravis, Guillain-Barré syndrome, and other inflammatory diseases of the nervous system; psoriasis, pemphigus vulgaris and other diseases of the skin; systemic lupus erythematosus, glomerulonephritis and other diseases affecting the kidneys; atherosclerosis and other inflammations of the blood vessels; autoimmune hepatitis, inflammatory bowel diseases, pancreatitis, and other conditions of the gastrointestinal system; type 1 diabetes mellitus, autoimmune thyroiditis, and other diseases of the endocrine system.

**Patentansprüche**

**1.** Gesättigter oder cis-ungesättigter $C_{10}$-$C_{18}$-Fettalkohol zur Verwendung als Immunmodulator bei der Behandlung von immunologisch vermittelten chronischen oder akuten entzündlichen Erkrankungen, ausgewählt aus einer Autoimmunerkrankung, schweren Allergien, Asthma, Transplantatabstoßung, oder für die Behandlung chronischer degenerativer Erkrankungen, wie Alzheimer-Krankheit, und bei der Neuroprotektion, der Organregeneration, chronischen Geschwüren der Haut und Schizophrenie.

**2.** Ausführungsform nach Anspruch 1, wobei der Immunmodulator ein gesättigter $C_{10}$-$C_{18}$-Fettalkohol, wie Decylalkohol, Laurylalkohol, Myristylalkohol, Cetylalkohol und Stearylalkohol, ist.

**3.** Ausführungsform nach Anspruch 1, wobei der Immunmodulator ein cis-ungesättigter $C_{16}$-$C_{18}$-Fettalkohol, wie Oleylalkohol, Linoleylalkohol, $\gamma$-Linolenylalkohol und Linolenylalkohol, ist.

**4.** Ausführungsform nach Anspruch 1, wobei die Autoimmunerkrankung Multiple Sklerose oder ein arthritischer Zustand beim Menschen ist.

**5.** Ausführungsform nach Anspruch 4, wobei der arthritische Zustand beim Menschen aus rheumatoider Arthritis, reaktiver Arthritis mit Reiter-Syndrom, Morbus Bechterew und anderen durch das Immunsystem vermittelten Entzündungen der Gelenke ausgewählt ist.

**6.** Ausführungsform nach Anspruch 1, wobei es sich bei der immunologisch vermittelten entzündlichen Erkrankung um Myasthenia gravis, Guillain-Barré-Syndrom und andere entzündliche Erkrankungen des Nervensystems; Schuppenflechte, Pemphigus vulgaris und andere Erkrankungen der Haut; systemischen Lupus erythematodes, Glomerulonephritis und andere die Nieren beeinträchtigende Erkrankungen; Atherosklerose und andere Entzündungen der Blutgefäße; autoimmune Hepatitis, entzündliche Darmerkrankungen, Pankreatitis und andere Zustände des Magen-Darm-Systems; Diabetes mellitus Typ 1, Autoimmunthyroiditis; und andere Erkrankungen des endokrinen Systems handelt.

**Revendications**

**1.** Alcool gras en $C_{10}$ à $C_{18}$ saturé ou cis-insaturé à utiliser comme immunomodulateur dans le traitement de troubles inflammatoires chroniques ou aigus induits par voie immunologique, choisis parmi une maladie auto-immune, des allergies sévères, l'asthme, le rejet de greffe ou pour le traitement de maladies dégénératives chroniques telles que la maladie d'Alzheimer et dans la neuroprotection, la régénérescence des organes, les ulcères chroniques cutanés et la schizophrénie.

**2.** Mode de réalisation selon la revendication 1, dans lequel l'immunomodulateur est un alcool gras en $C_{10}$ à $C_{18}$ saturé tel que l'alcool décylique, l'alcool laurylique, l'alcool myristylique, l'alcool cétylique et l'alcool stéarylique.

**3.** Mode de réalisation selon la revendication 1, dans lequel l'immunomodulateur est un alcool gras en $C_{16}$ à $C_{18}$ cis-insaturé tel que l'alcool oléylique, l'alcool linoleylique, l'alcool $\gamma$-linolénylique et l'alcool linolénylique.

**4.** Mode de réalisation selon la revendication 1, dans lequel ladite maladie auto-immune est la sclérose en plaques ou une affection arthritique humaine.

**5.** Mode de réalisation selon la revendication 4, dans lequel ladite affection arthritique humaine est choisie parmi l'arthrite rhumatoïde, l'arthrite réactive avec le syndrome de Reiter, la spondylite ankylosante et autres inflammations des articulations induites par le système immunitaire.

**6.** Mode de réalisation selon la revendication 1, dans lequel ledit trouble inflammatoire induit par voie immunologique est la myasthénie gravis, le syndrome de Guillain-Barré et autres maladies inflammatoires du système nerveux ; le psoriasis, le pemphigus vulgaire et autres maladies cutanées ; le lupus érythémateux systémique, la glomérulonéphrite et autres maladies rénales ; l'athérosclérose et autres inflammations des vaisseaux sanguins ; l'hépatite auto-immune, les maladies intestinales inflammatoires, la pancréatite et autres affections du système gastro-intestinal ; le diabète sucré de type 1, la thyroïdie auto-immune et autres maladies du système endocrinien.

Fig. 1

Fig. 2

Fig. 3

## REFERENCES CITED IN THE DESCRIPTION

### Patent documents cited in the description

- WO 9816216 A
- US 6331568 B
- WO 9927924 A
- US 6280755 B
- WO 9738688 A
- US 6262119 B
- WO 9958122 A
- US 6365628 B
- US 5019383 A
- WO 9630057 A

### Non-patent literature cited in the description

- Remington's Pharmaceutical Science. Mack Publishing Company, 1985
- **Birk et al.** The 60kDa heat shock protein modulates allograft rejection. *Proc. Nat. Acad. Science USA.,* 1999, vol. 96, 5159-63
- **Calder, PC.** Immunoregulatory and anti-inflammatory effects of n-3 polyunsaturated fatty acids. *Braz. J. Med. Biol. Res.,* 1998, vol. 31, 467-90
- **Cohen, IR.** Tending Adam's Garden: Evolving the Cognitive Immune Self. Academic Press, 2000
- **Cohen, IR.** Discrimination and Dialogue in the Immune System. *Seminars in Immunology,* 2000, vol. 12, 215-219269-271321-323
- **Holoshitz, Y. et al.** Lines of T lymphocytes mediate or vaccinate against autoimmune arthritis. *Science,* 1983, vol. 219, 56
- **Jaffee, B.D. et al.** The effect of immunomodulating drugs on adjuvant-induced arthritis in Lewis rats. *Agents Actions,* 1989, vol. 27 (3-4), 344-6
- **Lorentzen J.C. et al.** Protracted, relapsing and demyelinating experimental autoimmune encephalomyelitis in DA rats immunized with syngeneic spinal cord and incomplete Freund's adjuvant. *J. Neuroimmunology,* 1995, vol. 63, 193-205
- **Pearson, CM.** Experimental models in rheumatoid disease. *Arthritis Rheum.,* 1964, vol. 7, 80
- **Pearson, CM.** Development of arthritis, periarthritis and periostitis in rats given adjuvant. *Proc. Soc. Exp. Biol. Med.,* 1956, vol. 91, 91
- **Pollock et al.** Pharmacokinetic analysis of cyclosporine in adjuvant arthritic rats. *Drug Metab. Dispos.,* 1989, vol. 17 (6), 595-9
- **Rodriguez, M.D. ; Gamez, R. ; Sanchez, M ; H. Garcia.** *J. Appl. Toxicol.,* 1998, vol. 18, 313-6
- **Rose, N.R. ; F.J. Twarog ; A.J. Crowe.** Murine thryoiditis: importance of adjuvant and mouse strain for the induction of thyroid lesions. *J. Immunol.,* 1971, vol. 106, 698
- **Schwartz, M. ; Cohen.IR.** Autoimmunity can benefit self-maintenance. *Immunol Today,* 2000, vol. 21, 265-8
- The Merck Index. Merck & Co. Inc, 2001